**Europäisches Patentamt**

**European Patent Office**

**Office européen des brevets**

(11) Veröffentlichungsnummer: **0 015 381**
A2

# EUROPÄISCHE PATENTANMELDUNG

(21) Anmeldenummer: 80100466.4

(22) Anmeldetag: 30.01.80

(51) Int. Cl.³: **A 61 K 9/22, A 61 K 9/26**

(30) Priorität: 05.02.79 DE 2904310

(43) Veröffentlichungstag der Anmeldung: 17.09.80
Patentblatt 80/19

(84) Benannte Vertragsstaaten: **AT BE CH DE FR GB IT LU NL SE**

(71) Anmelder: **BOEHRINGER MANNHEIM GMBH,
Sandhofer Strasse 112-132 Postfach 31 01 20,
D-6800 Mannheim 31-Waldhof (DE)**

(72) Erfinder: **Stemmle, Berthold, Dr.rer.nat.,
Beethovenstrasse 16, D-6832 Hockenheim (DE)**
Erfinder: **Rothe, Werner, Dr.rer.nat., Walldorfer
Strasse 42, D-6832 Hockenheim (DE)**
Erfinder: **Heinemann, Helmut, Dr.rer.nat.,
Häusserstrasse 55, D-6900 Heidelberg 1 (DE)**

(54) **Formlinge mit retardierter Wirkstofffreisetzung und Verfahren zu deren Herstellung.**

(57) Die Erfindung betrifft ein Verfahren zur Herstellung von Formlingen mit retardierter Wirkstofffreisetzung bestehend aus dem Wirkstoff, üblichen Hilfsstoffen und einem wachsartigen, schwerlöslichen Hilfsstoff, wobei die nach bekannten Methoden hergestellten Formlinge bei einer Temperatur von 2–40°C unterhalb des Tropfpunktes des wachsartigen Hilfsstoffes 2–24 Stunden getempert werden, und Formlinge, die nach diesem Verfahren hergestellt sind.

EP 0 015 381 A2

Boehringer Mannheim GmbH                    2247

Formlinge mit retardierter Wirkstofffreisetzung
und Verfahren zu deren Herstellung

Die vorliegende Erfindung betrifft Formlinge mit retardierter
Wirkstofffreisetzung und Verfahren zu deren Herstellung, wie es
in den Patentansprüchen beschrieben ist.

Tabletten und Dragees mit einer verzögerten Abgabe des
Wirkstoffes und Verfahren zu deren Herstellung sind bereits
bekannt (magensaftresistente Überzüge, beschichtete Wirkstoffgranulate, Bindung an Ionenaustauscher , Überführen des
Wirkstoffes in schwerlösliche Derivate). In vielen Fällen wird
auch die Einbettung des Wirkstoffes in ein schwer- oder unlösliches Gerüst (Matrix) - meist polymere Hilfsstoffe oder
Wachse - vorgenommen, aus dem dieser langsam freigesetzt und
resorbiert wird.

Zur Herstellung von Matrixformen wird der Hilfsstoff entweder in einem organischen Lösungsmittel gelöst und mit den übrigen Substanzen gemischt oder auf irgendeiner Herstellungsstufe auf eine Temperatur oberhalb seines Tropfpunktes erwärmt, um eine gleichmäßige Umhüllung der anderen Stoffe zu erzielen.

Verfahren zur Retardierung von Wirkstoffen durch Granulation mit einer organischen Lösung von retardierenden Hilfsstoffen sind ebenfalls bekannt. Bei Verwendung von organischen Lösungsmitteln sind jedoch aus Gründen der Gesundheit und des Umweltschutzes erhebliche technische Schutzvorrichtungen erforderlich, so daß solche Verfahren nur ungern angewendet werden.

Bekannt ist auch die Einarbeitung gut löslicher Wirkstoffe in die Schmelze eines wachsartigen Hilfsstoffes mit anschließender Erstarrung. Nach diesem Verfahren werden jedoch keine Granulen definierter Größe erhalten, so daß für die Weiterverarbeitung zu Tabletten zusätzlich ein Arbeitsschritt zur Granulatformung notwendig wird. Auch die Herstellung von "Tropfperlen", (DE-OS 19 52 304) aus einer vergießbaren Schmelze, bestehend aus Wirkstoff und Träger, ist bekannt.

./.

Ebenso bekannt ist die Umhüllung durch wachsartige Hilfsstoffe mit Hilfe der Schmelzgranulation im beheizbaren Mischer (US-PS 3,308,217), im Mischer, bei dem die erforderliche Schmelztemperatur der wachsartigen Hilfsstoffe durch Friktionswärme erzielt wird oder durch Schmelzgranulation im Wirbelbett. (DE-PS 21 27 683)

Ein Nachteil dieser Verfahren besteht darin, daß der Wachsanteil, bezogen auf die Gesamtmenge Wirkstoff + Hilfsstoffe, eine bestimmte obere Grenze nicht überschreiten darf, damit die Granulate nicht miteinander zu großen Klumpen verschmelzen. Gut lösliche Wirkstoffe in hohen Dosierungen, die über einen längeren Zeitraum retardiert werden sollen, können deshalb nach diesen Verfahren nur schwer verarbeitet werden.

Bestimmte Substanzen lassen sich aufgrund ihrer grob kristallinen Struktur nicht ungemahlen im Wirbelbett schmelzgranulieren. So ist z. B. zur Retardierung von Kaliumchlorid eine vorhergehende Mahlung erforderlich, damit sich ein Wirbelbett ausbilden kann. Ebenso können explosionsgefährliche oder temperaturempfindliche Wirkstoffe nicht durch Schmelzgranulation verarbeitet werden.

Die NL-PS 6614 357 beschreibt ein Verfahren zur Herstellung von Depot-Tabletten, bei dem ein Granulat des wirksamen Stoffes mit einem leicht schmelzbaren, im Magen- und Darmsaft schwerlöslichen Stoff in Pulverform vermischt und zu Tabletten verpreßt wird. Die fertigen Tabletten werden in einem Wirbelbett auf eine Temperatur erhitzt, bei der der schmelzbare Stoff flüssig wird und aufgrund der Kapillarkräfte sich gleichmäßig in den Hohlräumen der Tablette verteilt. Gleichzeitig wird als Schutzfilm eine Lösung von Methylcellulose in Chloroform-Methanol aufgesprüht, um ein Zusammenkleben der Preßlinge zu verhindern.

./.

Es wurde nun überraschenderweise gefunden, daß homogene Matrixformen auch entstehen, wenn man durch direktes Verpressen bei Raumtemperatur hergestellte Formlinge, die wachsartige Hilfsstoffe in partikulärer Verteilung enthalten, bei einer Temperatur deutlich unterhalb des Tropfpunktes des wachsartigen Hilfsstoffes tempert. Hierbei bildet sich eine homogene Matrix aus, ohne daß bei diesen Temperaturen auch die aufeinander gelagerten Preßlinge miteinander verkleben.

Die zur Herstellung eines solchen Preßlings erforderlichen Hilfsstoffe richten sich in Art und Menge nach der gewünschten Freisetzungsgeschwindigkeit des Wirkstoffes und unterliegen keiner Beschränkung, außer, daß die preßfertige Masse formbar sein muß. Der durch Temperung erzielbare Retardeffekt ist in hohem Maße abhängig von der Art und der Menge des verwendeten wachsartigen Hilfsstoffs, der Art der übrigen Hilfsstoffe, den Wirkstoffeigenschaften und der Dauer und Temperatur des Temperschrittes, so daß sich die Freisetzungsgeschindigkeit des Wirkstoffes praktisch beliebig einstellen läßt.

Als Formlinge kommen bevorzugt Tabletten und Dragees in Frage, jedoch lassen sich nach diesem Verfahren natürlich auch andere Formen, beispielsweise Suppositorien herstellen. Auch ein direktes Tempern eines unverpreßten Granulats, das beispielsweise in Gelatinekapseln abgefüllt werden soll, führt erfindungsgemäß zu einer Retardierung des enthaltenen Wirkstoffs. Des weiteren können auch wirkstofffreie Granulate mit erfindungsgemäßer Zusammensetzung getempert werden, in die besonders temperaturempfindliche Wirkstoffe nachträglich eingearbeitet werden können. Die Matrix wird beim Verpressen des nun wirkstoffhaltigen Granulats gebildet und bewirkt eine Retardierung des Wirkstoffs.
Das direkte Tempern eines Granulats ist auch dann vorteilhaft, wenn weitere Wirkstoffe der Kapselmasse zugefügt werden sollen, für die eine Retardierung nicht erwünscht ist. Ebenso kann die retardierende Granulattemperung für die Herstellung von Mehrschichttabletten oder -dragees zur Anwendung kommen, wenn neben der Retardschicht in einer Schicht nicht retardierte Wirkstoffe enthalten sein sollen.

Als wachsartige Hilfsstoffe werden vorzugsweise solche mit einem Schmelzpunkt zwischen 40 und 100° C eingesetzt, z. B. Hartwachs, Polyethylenglykol, Stearylalkohol oder hydriertes Rizinusöl.

Als Bindemittel kommt z. B. in Kombination mit Wachsen vorzugsweise Polyvinylpyrrolidon zum Einsatz, wodurch ein deutlich stärkerer Retardierungseffekt als bei Verwendung von Wachsen allein erzielt wird.

Art und Menge weiterer üblicher hydrophiler und hydrophober Hilfsstoffe richten sich nach der anzustrebenden Retardierungszeit.

Der Wirkstoff wird zusammen mit dem unlöslichen, schmelzbaren Hilfsstoff und gegebenenfalls weiteren Hilfsstoffen durch Zugabe einer Granulierflüssigkeit, gegebenenfalls mit einem Bindemittel wie üblich granuliert.

Neben der feuchten Granulation können auch übliche trockene Granulierverfahren angewendet werden. Die Granulate werden mit weiteren Tablettierhilfsstoffen versetzt und zu Tabletten verpreßt.

In günstigen Fällen ist auch eine trockene Tablettierung durch einfache Vermischung des Wirkstoffes mit dem schmelzbaren Hilfsstoff und weiteren Zuschlagsstoffen möglich.

Nach der Verpressung werden die Formlinge ca. 2 - 24 Stunden auf eine Temperatur deutlich unterhalb des Tropfpunktes des schmelzbaren Hilfsstoffes erwärmt, d. h. auf Temperaturen von 2 - 40° C, vorzugsweise 10 - 20° C unterhalb des Tropfpunktes, aber mindestens 20 - 40° C oberhalb der Raumtemperatur. So genügt hierfür z. B. für ein Hartwachs mit einem Tropfpunkt von 79 - 82° C eine Temperatur von 45 - 65° C. Unter Tropfpunkt wird dabei diejenige Temperatur verstanden, bei der der Stoff unter seinem Eigengewicht von einer Thermometerkugel oder dem Tropfnippel einer entsprechenden Meßvorrichtung abtropft und der ungefähr dem Fließpunkt der Substanz entspricht.

Anhand der folgenden Beispiele soll das erfindungsgemäße Verfahren erläutert werden.

## Beispiel 1

| | 1 Stück | Ansatz für 45.000 Stück |
|---|---|---|
| **Rezeptur:** | | |
| I   Kaliumchlorid, krist. | 300 mg | 13,500 kg |
|      Hartwachs | 30 mg | 1,350 kg |
|      Polyvinylpyrrolidon | 15 mg | 0,675 kg |
|      (mittl. Mol.-Gew. 25.000) | | |
| II   Magnesiumstearat | 5 mg | 0,225 kg |

### Verarbeitung

Die Bestandteile aus I werden gesiebt, gemischt und mit Wasser granuliert, getrocknet und gesiebt. Das gesiebte Granulat wird mit II homogen gemischt und tablettiert. Die Preßlinge werden ca. 24 Stunden bei 60° C getempert.

### In-vitro-Auflösungsrate von KCl

| Zeit (min) | 10 | 20 | 30 | 60 | 90 | 120 | 150 | 180 |
|---|---|---|---|---|---|---|---|---|
| Gelöstes KCl (%) (getempert) | 26 | 39 | 46 | 59 | 67 | 76 | 81 | 87 |
| Gelöstes KCl (%) (ungetempert) | 60 | 65 | 75 | 97 | | | | |

## Beispiel 2

| | 1 Stück | Ansatz für 50.000 Stück |
|---|---|---|
| **Rezeptur:** | | |
| Kaliumchlorid, krist. | 300 mg | 15,000 kg |
| Hartwachs | 50 mg | 2,500 kg |
| Polyvinylpyrrolidon | 15 mg | 0,750 kg |
| (mittl. Mol.-Gew. 25.000) | | |
| Magnesiumstearat | 5 mg | 0,250 kg |

Verarbeitung

Alle Bestandteile der Rezeptur werden gesiebt, homogen gemischt und tablettiert. Die Preßlinge werden 24 Stunden bei 60° C getempert.

In-vitro-Auflösungsrate von KCl

| Zeit (min) | 10 | 20 | 30 | 60 | 90 | 120 | 150 | 180 | 210 | 240 |
|---|---|---|---|---|---|---|---|---|---|---|
| Gel. KCl (%) (getempert) | 27 | 36 | 41 | 52 | 62 | 69 | 76 | 81 | 86 | 90 |
| Gel. KCl (%) (ungetempert) | 48 | 64 | 74 | 92 | | | | | | |

## Beispiel 3

| | | 1 Stück | Ansatz für 10.000 Stück |
|---|---|---|---|
| Rezeptur: | | | |
| I | Isosorbiddinitrat | 20,0 mg | 200,0 g |
| | Lactose | 123,5 mg | 1235,0 g |
| | Hartwachs | 15,0 mg | 150,0 g |
| | Talkum | 25,0 mg | 250,0 g |
| II | Kieselsäure, hochdispers | 1,5 mg | 15,0 g |
| | Magnesiumstearat | 5,0 mg | 50,0 g |

Verarbeitung

Die Bestandteile aus I werden gemischt, mit Wasser granuliert, getrocknet und gesiebt. Das gesiebte Granulat wird mit II homogen gemischt und tablettiert. Die Preßlinge werden ca. 2 Stunden bei 60° C getempert.

./.

## In-vitro-Auflösungsrate von ISDN

| Zeit (Stunden) | 2 | 3 | 4 |
|---|---|---|---|
| Gelöstes ISDN (%) (getempert) | 56 | 88 | 98 |
| Gelöstes ISDN (%) (ungetempert) | 95 | | |

## Beispiel 4

|  | | 1 Stück | Ansatz für 10.000 Stück |
|---|---|---|---|
| Rezeptur: | | | |
| I | Metipranolol | 40,0 mg | 400,0 g |
| | Lactose K Ph. Eur. | 31,0 mg | 310,0 g |
| | Hartwachs | 10,0 mg | 100,0 g |
| | Polyvinylpyrrolidon (mittl. Mol.-Gew. 25.000) | 5,0 mg | 50,0 g |
| II | Aerosil 200 | 1,0 mg | 10,0 g |
| | Magnesiumstearat | 3,0 mg | 30,0 g |

## Verarbeitung

Die Bestandteile aus I werden gesiebt, gemischt und mit Wasser granuliert, anschließend getrocknet und gesiebt. Das gesiebte Granulat wird mit II homogen gemischt und tablettiert. Die Preßlinge werden 24 Stunden bei 60° C getempert.

## In-vitro-Auflösungsrate von Metipranolol

| Zeit (Stunden) | 0,5 | 1 | 2 | 3 | 4 |
|---|---|---|---|---|---|
| Gelöstes Metipranolol (%) (getempert) | 24 | 36 | 50 | 60 | 70 |
| Gelöstes Metipranolol (%) (ungetempert) | 43 | 70 | 90 | | |

Beispiel 5

|                    | 1 Stück   | Ansatz für 10.000 Stück |
|--------------------|-----------|-------------------------|
| Rezeptur:          |           |                         |
| Nitrofurantoin, feinkristallin | 100,0 mg | 1.000,0 g |
| Polyethylenglykol 5/6000 Pulver | 30,0 mg | 300,0 g |

Verarbeitung

Die Bestandteile werden gesiebt, gemischt und mit Wasser granuliert, anschließend bei 45° C getrocknet, wobei gleichzeitig die Temperung des Granulats erfolgt. Das getemperte Granulat wird gesiebt und entweder allein oder gegebenenfalls mit weiteren üblichen Hilfsstoffen zusammen in Gelatinekapseln abgefüllt.

In-vitro-Auflösungsrate von Nitrofurantoin

| Zeit (Stunden) | 0,5 | 1 | 2 | 3 | 4 | 5 |
|---|---|---|---|---|---|---|
| Gelöstes Nitrofurantoin (%) (Granulat getempert) | 29 | 33,5 | 52,5 | 62 | 69,5 | 76 |
| Gelöstes Nitrofurantoin (%) (Mischung) | 100 | | | | | |

## P a t e n t a n s p r ü c h e

1. Verfahren zur Herstellung von Formlingen mit retardierter Wirkstofffreisetzung bestehend aus dem Wirkstoff, üblichen Hilfsstoffen und einem wachsartigen, schwerlöslichen Hilfsstoff, dadurch gekennzeichnet, daß die nach bekannten Methoden hergestellten Formlinge bei einer Temperatur von 2 - 40° C unterhalb des Tropfpunktes des wachsartigen Hilfsstoffs 2 - 24 Stunden getempert werden.

2. Verfahren nach Anspruch 1, dadurch gekennzeichnet, daß bei einer Temperatur von 10 - 20° C unterhalb des Tropfpunktes getempert wird.

3. Formlinge, hergestellt nach dem Verfahren gemäß Anspruch 1 oder 2.